# EUROPEAN PATENT APPLICATION

(11) **EP 2 409 982 A2**
(43) Date of publication of application: **25.01.2012**
(21) Application number: 10305190.0
(22) Date of filing: 25.02.2010
(51) Int. Cl.: C07K 1/02

(54) **Chemical processes generating solid(s) carried out continuously within microreactors**

(71) Applicant: Corning Incorporated, Corning NY 14831 (US)
(72) Inventor: Van Zutphen, Steven, 77780, Bourron Marlotte (FR); Zhang, Feixia, 77920, Samois-sur-Seine (FR)
(74) Representative: Le Roux, Martine

(57) **Abstract**

The present invention relates to a continuous process for conducting a chemical reaction in a liquid reaction medium. Characteristically:
- said continuous process is carried out in a microreactor (10),
- said chemical reaction produces a solid insoluble in said liquid reaction medium, and
- said continuous process comprises the continuous extraction of said solid, as it is produced, with a solvent S2, immiscible with said reaction medium.

Such a process is advantageously carried out for conducting the activation of a carboxylic acid in the presence of an organic base.

## Description

The present invention concerns a continuous process for conducting a chemical reaction in a liquid reaction medium. It more particularly concerns such a continuous process carried out in a microreactor for conducting a chemical reaction which produces a solid (precipitate) insoluble in said liquid reaction medium, while avoiding plug formation.

### BACKGROUND OF THE INVENTION

Many chemical reactions generate solids (precipitates), insoluble in the reaction medium. To carry out such reactions in a continuous-flow setup can be challenging as the solid inside the channel may have a tendency to coagulate and block the channel. This will effectively stop the continuous flow.

A typical reaction where a solid (precipitate) is formed is amide bond forming reaction in peptide synthesis, as it is industrially carried out up to now. In this reaction the carboxylic acid of a first amino acid reacts with the amine of a second amino acid. The reaction is a two step process. In the first step the carboxylic acid needs to be activated, to form, typically, an activated ester. In organic solvent, this is carried out in the presence of an organic base, such as triethylamine. During this step, a water soluble precipitate (salt) is formed in a reaction, which is typically performed in organic solvent, as both the reagent used to activate the acid as well as the activated ester formed are sensitive to water. In the second step of the coupling reaction the activated ester will go on to react with the amine of the second amino acid to form the desired peptide. Such an amide bound formation reaction is found in the fabrication of many pharmaceutically relevant molecules.

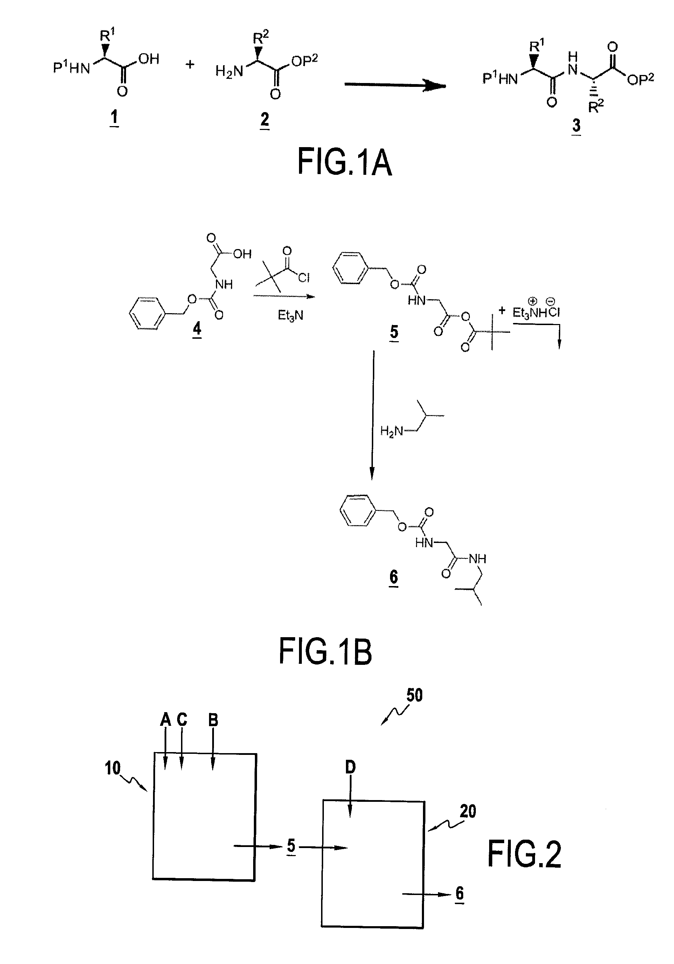

Such an amide bound formation reaction is generally schematized in attached figure 1A (the dipeptide (**3**) is the product of an amine coupling reaction between an activated ester of the first amino acid (**1**) and an amine function of the second amino acid (**2**) in the presence of a base (2eq.) and of an activating agent (1 eq.)). A specific example of such a reaction - the one carried out according to the examples of this application - is schematized in attached figure 1B (Z-Gly-OH (**4**) is activated with pivaloyl chloride in the presence of triethylamine to give the activated ester (**5**). This then reacts with iso-butylamine to yield the final product (**6**)).

Generally speaking, removing solids from a reaction medium can be done either by extraction or by filtration.

In reference to the traditional chemical processes, the following may be indicated. In batch reactions where solid precipitates are formed, filtration is generally used. Alternatively, extraction with a second solvent that is immiscible with the reaction medium (containing a first solvent), but capable of dissolving the precipitate can be done. In a continuous-flow process filtration can be difficult to implement as the filter gradually saturates. This implies that a regeneration step must somehow be implemented. Continuous extraction can then be an attractive alternative. Liquid-liquid continuous extractions have more particularly been described in Perry's Chemical Engineers' Handbook (8th Edition), section 15.10, 2008. Extractions carried out simultaneously with the reaction can be particularly advantageous for the purposes of process intensification. Such extractions - reactive separations - can be carried out, for example, to drive equilibrium reactions or to improve catalytic processes ("Process intensification, a path to the future", Charpentier, J.C., 2006, Ingenieria Quimica, 38 (434), pp. 16-24).

To the inventors' knowledge, prior art is silent about continuous extraction or reactive separation carried out within microreactors. No doubt that a serious technical prejudice exists in reference to the severe requirements: finding solvent/non solvent couples, the non solvent of which is effective, within microchannels, with regard to the extraction, so as to avoid any formation of plugs inside said microchannels (such plugs being able to clog said microchannels), without being (too) detrimental with regard to the reaction carried out.

To come back to the context of the peptide synthesis mentioned above, the following may be added. If the synthesis is carried out according to a batch process (Tetrahedron 44(6), 1685-1690, 1988), the salt (precipitate) formed in the aforementioned first step, will be removed by filtration. If the synthesis (more particularly its first step, related to the activation of the carboxylic acid) is carried out according to a continuous process, for example in a microchannel, the salt can quickly block the channel. Then there are several ways to avoid (and not to solve) this problem. According to the prior art, it has more particularly been proposed:
1) to replace the common base with a more hydrophobic and higher boiling point base, such as tributylamine. Then the salt formed remains soluble in organic solvent. Removing the base from the products now becomes a problem;
2) an other alternative method can be the use of very dilute conditions in a solvent such as DMF. In these conditions organic salts can remain in solution. However this method uses large amounts of toxic solvent;
3) still another alternative can be the use of activating agents which does not imply the formation of any precipitate in the reaction medium. Such activating agents are expensive and are not used industrially on large scale. Their use is mentioned in patent application EP-A-1 801 086.

So the above prior art proposed solutions to the technical problem of clogging the microchannels by plug formation are not totally satisfactory. In any way, they are all based on modifications of the reaction conditions in view of avoiding the problem, in view of avoiding any formation of precipitate. They tackle the problem preventatively The prior art has neither disclosed nor suggested any possibility for carrying out the above described amide bound formation reaction with generation of a precipitate in a continuous way within a microreactor (thus, confirming the above mentioned prejudice).

### THE INVENTION

In such a context, the inventors propose a continuous process for conducting a chemical reaction in a liquid reaction medium. Said continuous process shows the three below characteristic technical features, in combination:
- it is carried out in a microreactor,
- it is carried out for conducting a chemical reaction which produces a (it has to be read at least one) solid (precipitate) insoluble in the liquid reaction medium; and
- it comprises the continuous extraction of said (at least one) solid (precipitate), as it is produced, with a solvent S2, immiscible with said reaction medium.

In the following, "a solid" is "at least one solid". It is generally one solid. If different solids are able to be generated, the solvent S2 has to be able to extract said different solids.

So, according to the invention, the solid (precipitate) formed during a chemical process carried out in a continuous flow within a microreactor (inside channels of small dimensions; hence the criticity of the clogging problem with said solid (precipitate)) is characteristically extracted continuously and simultaneously. Said solid or precipitate is extracted as it is produced. It is removed as the reaction proceeds within the microreactor. This approach allows solid (precipitate) forming reactions to be carried out efficiently in channel of microreactors without risk of channel blocking.

This approach is novel insofar as it prevents the clogging problem not in avoiding the forming of a solid (a precipitate) (see above, the concept of providing solution preventatively) but in managing said formation of a solid (a precipitate): in eliminating the formed solid (or precipitate) as soon as it is formed. Said elimination occurs through an extraction process using a suitable solvent: S2, able to (selectively) extract said solid or precipitate and immiscible with the reaction medium.

This approach is against the above prejudice: it is possible to carry out efficient solvent extraction within channels of small dimensions; it is possible to carry out such efficient solvent extraction without serious modification of the conditions of reactions and/or deterioration of the results. The inventors have shown that the process of the invention allows performing chemical reactions in a continuous flow channel set up while using the same chemicals and the same solvents as what is commonly used in some prior art batch processes.

The man skilled in the art has up to now understood how interesting the process of the invention is.

The advantages are thus firstly all the advantages that a continuous-flow process can bring such as flexible scaling, precise stoechiometric control and controlled heat management. Secondly, with regards to other alternatives carried out in a continuous-flow setup (see above), the advantages of the process of the invention include possibility of using cheap and common reagents, and workup methods already optimized for this kind of reaction.

The process of the invention may consist in a transposition of a known batch process, the formed solid being extracted continuously (instead of being filtered off at the end of the batch process). It may also consist in an amended transposition, where the couple solvent of reaction medium and S2 are optimized to ensure good selective transfer of the formed precipitate to S2, but avoiding any reagents to be deactivated by S2.

It has to be noted that the solid (precipitate) formed inside the microreactor and immediately extracted with the solvent S2 (dissolved in said solvent S2) may be a product of the conducted chemical reaction or a by-product of said chemical reaction. As a product, it may be a final product or an intermediate product. It is actually not impossible to contemplate a further reaction of said extracted product within the solvent S2.

S2 is advantageously selected to conduct an extraction as selective as possible, to perturb, not at all, at minima, the conducted chemical reaction. The man skilled in the art is able to optimize the nature of the extraction solvent S2 in any context.

Concerning the general context of microreactors, the following may be added.

Microreactors or microfluidic devices are known for a long time and may be obtained from different processes, more particularly from those described in patent applications US 2004/0206391 or WO 2008/106160. The dimension(s) of their channels have actually increased with time and now the term of "microreactor" is issued for both microreactor with microchannels (1-100 µm) and "microreactor" with channels of greater size (100 µm - a few mm). The channels or microchannels may show sections of different forms, for example circular, square, rectangular.

The process of the invention is carried out in microreactors, i.e. in reactors having channels smaller than the pipes of the traditional chemical processes. Generally, the process of the invention is carried out in a microreactor with channels having an equivalent diameter within the range of 1 µm - 1 cm.

Advantageously, it is carried out in a microreactor with channels having an equivalent diameter within the range of 0.5 mm - 4 mm.

Surprisingly, as indicated above, efficient extractions are carried out according to the process of the invention inside such channels.

The extraction solvent S2 is suitable to extract (to dissolve) the solid (the precipitate) as soon as it forms. Depending on the nature of said solid (and of the nature of the liquid reaction medium), the nature of S2 is determined, is advantageously selected.

Extraction solvent S2 is generally selected from the group consisting in water, advantageously water containing at least one salt, alcohols, toluene, ethyl acetate, dichloromethane, heptane, hexane, cyclohexane and fluorinated solvents.

The presence of at least one salt in water may be opportune to have better phase separation between S2 and the reaction medium.

Concerning the liquid reaction medium, it may include a solvent S1 or it may be free of any solvent. In this latter case, the reactive medium is *per se* liquid.

Most of chemical reactions are carried out in a solvent and so the process of the invention is then carried out with two solvents S1 and S2, the extraction solvent S2 being immiscible with S1. The two possibilities exist: S1 is a non-polar solvent while S2 is a polar solvent OR S1 is a polar solvent while S2 is a non-polar solvent.

Many chemical reactions are actually carried out in organic solvents. So the process of the invention, when carried out with a solvent S1, is generally carried out with a non-polar solvent S1, with such a non-polar solvent S1 advantageously selected from the group consisting in toluene, ethyl acetate, dichloromethane, heptane, hexane, cyclohexane, and fluorinated solvents.

So, according to a preferred variant, the process of the invention is carried out with an extraction solvent S2 selected from the group consisting in water, advantageously water containing at least one salt, and alcohols.

Said solvent S2 is very preferably water, advantageously water containing at least one salt.

Said solvent S2 is very preferably water containing NaCI.

According to a preferred variant, the continuous process of the invention is carried out with a liquid reaction medium including a solvent S1 consisting in toluene, ethyl acetate or dichloromethane, advantageously in ethyl acetate and an extraction solvent S2 consisting in water or water containing at least one salt, advantageously in water containing at least one salt.

Within said preferred variant, it is particularly advantageous that said liquid reaction medium includes a solvent S1 consisting in ethyl acetate and said extraction solvent S2 consists in water containing NaCI.

The use of said couple S1/S2 is particularly interesting in reference to the chemical reaction consisting in the activation of a carboxylic acid in the presence of an organic base (see later as well as the filed examples and attached figures).

The process of the invention may exist *per se,* related to a single chemical reaction, carried out in a microreactor or in several microreactors, arranged in series or in parallel.

It may also exist as part of a general process for conducting the chemical reaction (generating a solid) and at least one additional chemical reaction carried out downstream from said chemical reaction, said chemical reaction and said at least one additional chemical reaction being carried out in at least two microreactors arranged in series.

Obviously:
- it is not all excluded that it may also exist as part of a general process comprising at least one additional chemical reaction carried out upstream;
- it is not at all excluded that it may also exist as part of a general process comprising at least one additional chemical reaction carried out upstream and at least one additional chemical reaction carried out downstream; (all reactions being carried out in microreactors arranged in series).

The process of the invention carried out for conducting the single chemical reaction of a process or for conducting one chemical reaction of a general process including at least two chemical reactions generally further includes a liquid-liquid separation step where the reaction medium (without solvent or with solvent S1) and S2 are physically separated in a batch or continuous way.

There exist many contexts and several ways of carrying out the process of the invention.

It is particularly advantageous to carry out the process of the invention for conducting the chemical reaction consisting in the activation of a carboxylic acid in the presence of an organic base (the generated solid (precipitate) then consisting in the salt of said organic base).

The invention can thus be localized in the peptide synthesis context (see the introducing part of the specification as well as the following examples and attached figures). The man skilled in the art understands that it is in no case limited to said context.

In a non limitative way, it may be indicated that:
- the organic base is advantageously isobutylamine, triethylamine, ethyldiisopropyl amine or N-methylmorpholine;
- the activation reactant is advantageously an acid chloride such as pivaloyl chloride, isobutylchloroformate or ethylchloroformate.

Such a chemical reaction is advantageously carried out according to the invention with the couple S1/S2, as indicated above, more particularly :
(S1/S2) = (toluene/water) = (ethyl acetate/NaCl containing water).

In such a context, the process of the invention is advantageously part of a two-step peptide coupling (synthesis) process:
- the activation being carried out according to the invention in a first microreactor,
- the coupling being carried out downstream in a second microreactor.

The coupling (actually any chemical reaction carried out after the use of S2) is carried out within a phase (the one containing S2) of a biphasic liquid. It is advantageously carried out with mixing, for example inside a microreactor including static mixers.

The claimed invention is now illustrated, in a non limitative way, by the following examples and annexed figures.

### FIGURES

Figure 1A shows the general reaction scheme for an amide bond forming reaction.
Figure 1B shows the specific amide bond forming reaction which is carried out in the following examples 1 and 2.
Figure 2 schematically shows a device suitable for carrying out said specific amide bond forming reaction, with implementation of the process of the invention (within the first microreactor).

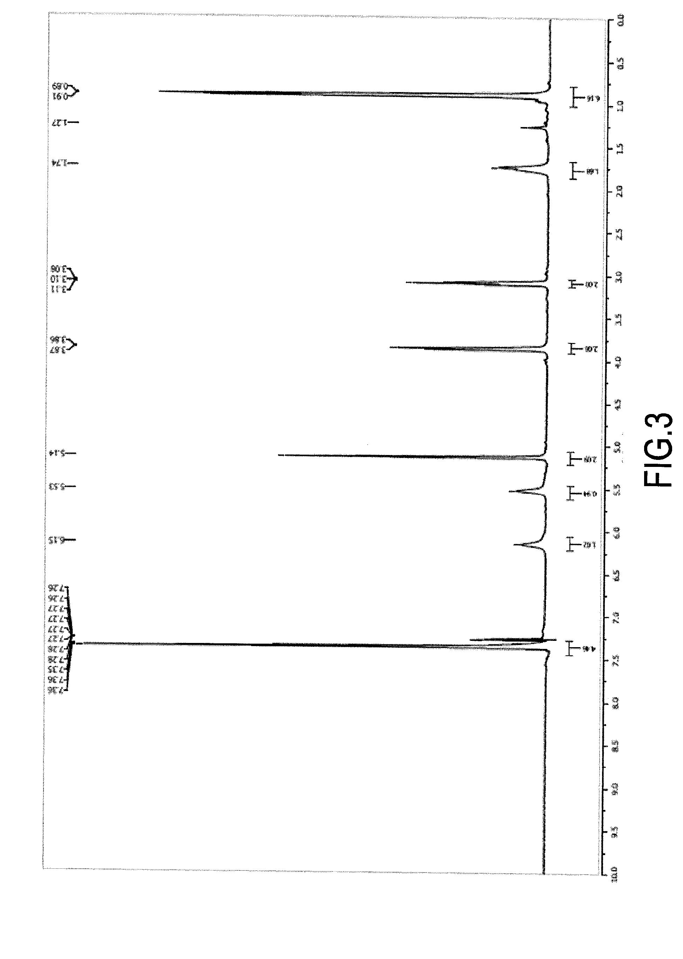

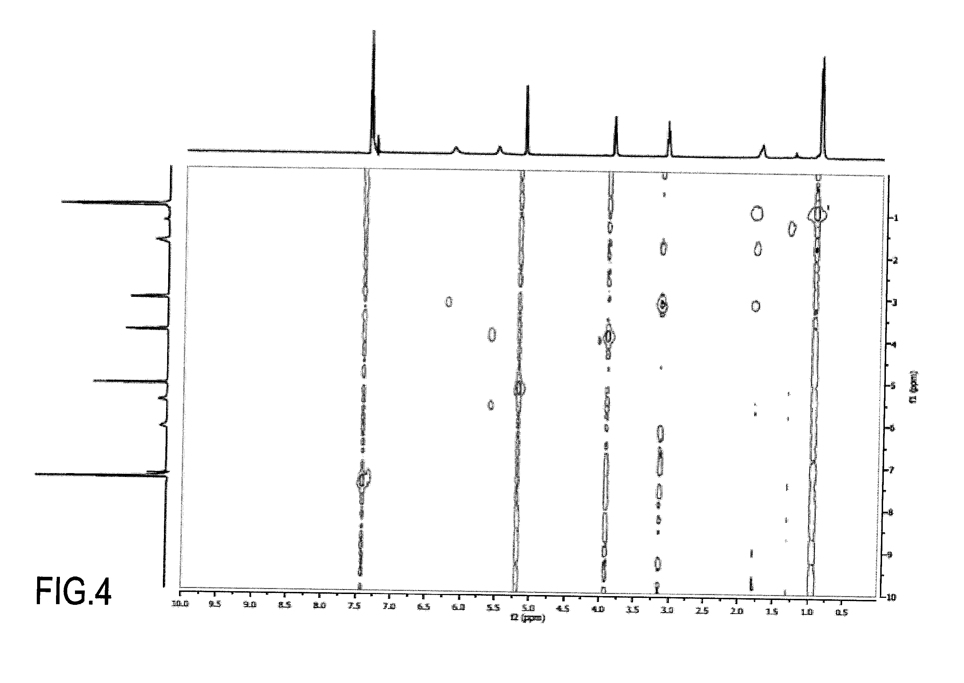

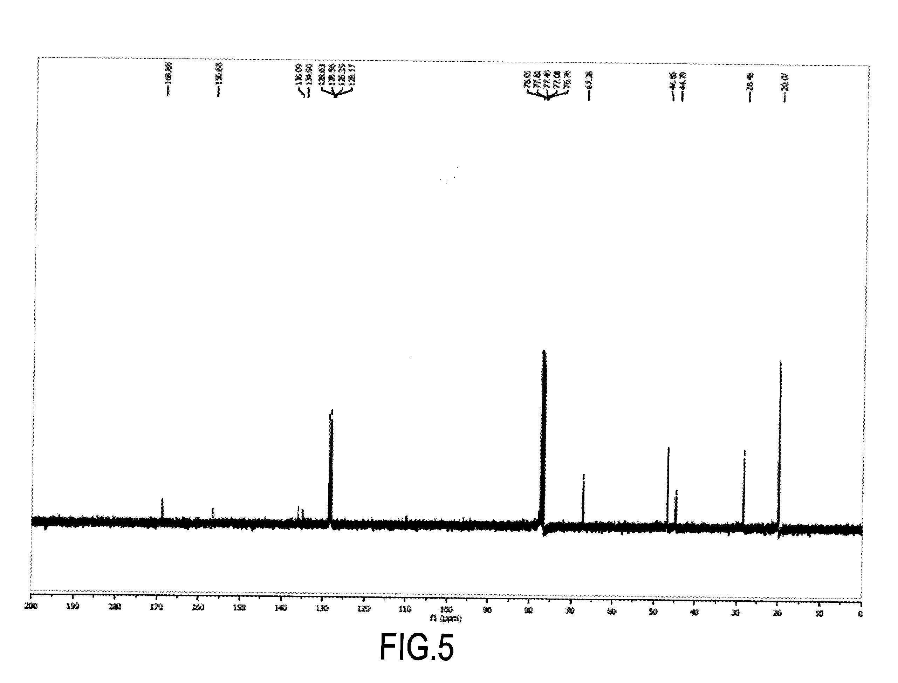

Figures 3, 4 and 5 respectively show the ¹HNMR spectrum, the gCOSY NMR spectrum and the ¹³C NMR spectrum of compound (**6**) (peptide obtained according to the reaction of figure 1B, according to the following examples (more particularly according to following example 2)).

Figure 1A and 1B have been commented above, in a paragraph included in the part of the specification entitled "Background of the invention".

So the two-step reaction shown in Figure 1B is the one carried out in the following examples 1 and 2.

Said reaction is carried out in the device 50 schematically represented in Figure 2. Said device 50 comprises two microreactors 10, 20 which are arranged in series.

Within the first microreactor 10, the activated ester (**5**) is prepared. The raw solutions A (A1 in example 1, A2 in example 2), B (B1 in example 1, B2 in example 2) and C (C1 in example 1, C2 in example 2) are described in the following examples. The first microreactor 10 is a straight channel flow-reactor. A mixing fluidic device is not required to carry out the activation of the carboxylic acid (**4**).

Within the second microreactor 20, the activated ester (**5**) is reacted with the amine. The raw solution D (D1 in example 1, D2 in example 2), including said amine in a solvent, is described in the following examples. The second microreactor 20 is a mixing reactor, is actually (according to the examples) a flow-reactor containing static mixers, as described in patent application EP-A-2 017 000. The mixing carried out is particularly opportune insofar as the reaction occurs in a biphasic reaction medium (S1/S2: see the below examples).

Concerning the NMR spectra of the prepared peptide (6), we may precise the following.

| - ¹H NMR : | Parameter | Value |
|---|---|---|
| | 1. Solvent | CDCl₃ |
| | 2. Temperature | 20°C |
| | 3. Number of scans | 8 |
| | 4. Nucleus | 1H |

¹H NMR (400 MHz, CDCl₃) δ 7.35 (m, 5H, Ph), 6.15 (m, 1H, OCON*H*), 5.53 (m, 1H, CH₂CON*H*), 5.14 (m, 2H, PhC*H₂*), 3.87 (m, 2H, αCH₂), 3.10 (m, 2H, C*H₂*CH), 1.74 (m, 1H, CH), 0.90 (m, 6H, CH₃).

| - gCOSY NMR | Parameter | Value |
|---|---|---|
| | 1. Solvent | CDCl₃ |
| | 2. Pulse sequence | gCOSY |
| | 3. Temperature | 20°C |
| | 4. Number of scans | 1 |
| | 5. Nucleus | (1H,1H) |

| - ¹³C NMR | Parameter | Value |
|---|---|---|
| | 1. Solvent | CDCl₃ |
| | 2. Temperature | 20°C |
| | 3. Number of scans | 1000 |
| | 4. Nucleus | ¹³C |

¹³C NMR (101 MHz, CDCl₃) δ 168.88 (CO), 156.68 (CO), 136.09-128.17 (Ph), 67.28 (*C*H₂Ph) , 46.85 (αC), 44.79 (*C*H₂CH), 28.48 (CH), 20.07 (CH₃).

These spectra clearly confirm the chemical structure of said compound (**6**).

### EXAMPLES

### Preparation of solutions

### EXAMPLE 1

Solution A1: 10.5 g Z-Gly-OH and 32.1 ml triethylamine dissolved in 93 ml of toluene (0.4 M amino acid)

Solution B1: 7.4 ml trimethylacetyl (pivaloyl) chloride in 143 ml of toluene (0.4 M)

Solution C1: 200 ml water

Solution D1: 12 ml iso-butylamine in 200 ml of toluene (0.6 M).

In said example 1, S1 = toluene while S2 = water.

### EXAMPLE 2

Solution A2: 10.5 g Z-Gly-OH and 32.1 ml triethylamine dissolved in 93 ml of ethyl acetate (0.4 M amino acid)

Solution B2: 7.4 ml trimethylacetyl (pivaloyl) chloride in 143 ml of ethyl acetate (0.4 M)

Solution C2: 200 ml water containing 10 wt% NaCl

Solution D2: 12 ml iso-butylamine in 200 ml ethyl acetate (0.6 M).

In said example 2, S1 = ethyl acetate while S2 = NaCl containing water.

### Flow-rates and experimental setup

Solution A (A1 (example 1) and A2 (example 2)) and Solution B (B1 and B2, respectively) were pumped each with a flow rate of 10 g/min along with solution C (C1 and C2 respectively) at a flow rate of 15 g/min into a non-mixing fluidic module 10 kept at a temperature of 5-10 °C. In this module 10 the activated ester of the Z-Gly is formed. The reaction was then quenched in a second, mixing fluidic module 20 by solution D (D1 and D2 respectively) introduced at a flow rate of 15 g/min. The organic phase was separated, washed with 1M HCI and dried with MgSO₄. The solvent (S1 = toluene in example 1, S1 = ethyl acetate in example 2) was evaporated under reduced pressure and the resulting solid (product (6)) was purified over a silica column (gradient cyclohexane 100% to ethylacetate 100%) and characterized fully using a VARIAN 400MR NMR spectrometer (the attached spectra are those of the product of example 2) Product (6) is obtained with about 30 % yield after column for example 1; 63% yield after column for example 2).

The channels of the microreactors 10 and 20 have an equivalent diameter of 1 mm.

### Comments

In the experiments it was clearly observed that the water (S2 = H₂O or H₂O + NaCl) allowed the reaction to run for long periods of time. Over runs of 20 min or more, no increase in the back pressure of the microreactors was observed.

Contrarily, when no water (no S2) was added to the reaction medium, the pressure instantly rose, and the pumps switched off automatically within 2 min from starting the run, leaving the microreactor clogged.

Initially the reactivity of the pivaloyl chloride with water was feared to be a limiting factor for this reaction. However, since this reagent is in the organic layer, it did not have much contact with the water.

The main by-product of the reaction was in fact the product of pivaloyl chloride with iso-butylamine, clearly demonstrating that the acid chloride was not rapidly deactivated by the water.

The solubility of the (deprotonated) acid is a limiting factor in this reaction. If the acid is extracted into the water before the activation reaction takes place, the final yield will be very low. So extraction of said acid is opportunely limited by introducing NaClin the water and by choosing a solvent that dissolved the amino acid best. Introducing NaCl and changing the solvent from toluene to ethyl acetate, the purified yield of the desired product could be increased from about 30% to about 60% purified (see above). The above examples 1 and 2 both illustrate the claimed invention. Example 2 illustrates an advantageous variant.

## Claims

1. A continuous process for conducting a chemical reaction in a liquid reaction medium, **characterized in that**:
- said continuous process is carried out in a microreactor (10),
- said chemical reaction produces a solid insoluble in said liquid reaction medium, and
- said continuous process comprises the continuous extraction of said solid, as it is produced, with a solvent S2, immiscible with said reaction medium.

2. The continuous process according to claim 1, **characterized in that** it is carried out in a microreactor (10) with channels having an equivalent diameter within the range of 1 µm - 1 cm, advantageously within the range of 0.5 mm - 4 mm.

3. The continuous process according to claim 1 or 2, **characterized in that** said solvent S2 is selected from the group consisting in water, advantageously water containing at least one salt, alcohols, toluene, ethyl acetate, dichloromethane, heptane, hexane, cyclohexane and fluorinated solvents.

4. The continuous process according to any one of claims 1 to 3, **characterized in that** said liquid reaction medium includes a solvent S1 or includes no solvent.

5. The continuous process according to any one of claims 1 to 4, **characterized in that** said liquid reaction medium includes a solvent S1 ; S1 being a non-polar solvent while S2 being a polar solvent or S1 being a polar solvent while S2 being a non-polar solvent.

6. The continuous process according to any one of claims 1 to 5, **characterized in that** said liquid reaction medium includes a solvent S1 ; S1 being selected from the group consisting in toluene, ethyl acetate, dichloromethane, heptane, hexane, cyclohexane, and fluorinated solvents.

7. The continuous process according to any one of claims 1 to 6, **characterized in that** said solvent S2 is selected from the group consisting in water, advantageously water containing at least one salt, and alcohols.

8. The continuous process according to any one of claims 1 to 7, **characterized in that** said solvent S2 is water, advantageously water containing at least one salt.

9. The continuous process according to any one of claims 1 to 8, **characterized in that** said solvent S2 is water containing NaCl.

10. The continuous process according to any one of claims 1 to 9, **characterized in that** said liquid reaction medium includes a solvent S1 consisting in toluene, ethyl acetate or dicloromethane, advantageously in ethyl acetate and S2 consists in water or water containing at least one salt, advantageously in water containing at least one salt.

11. The continuous process according to any one of claims 1 to 10, **characterized in that** said liquid reaction medium includes a solvent S1 consisting in ethyl acetate and S2 consists in water containing NaCl.

12. The continuous process according to any one of claims 1 to 11, **characterized in that** it is part of a general process for conducting said chemical reaction and at least one additional chemical reaction carried out upstream and/or downstream from said chemical reaction, said chemical reaction and said at least one additional chemical reaction being carried out in at least two microreactors (10, 20) arranged in series.

13. The continuous process according to any one of claims 1 to 12, **characterized in that** said chemical reaction consists in the activation of a carboxylic acid in the presence of an organic base.

14. The continuous process according to claim 13, **characterized in that** it is part of a two step peptide coupling process.
